Europäisches Patentamt

European Patent Office

Office européen des brevets

(11) Numéro de publication : **0 434 485 B1**

# (12) FASCICULE DE BREVET EUROPEEN

(45) Date de publication du fascicule du brevet :
**29.12.93 Bulletin 93/52**

(51) Int. Cl.⁵ : **C07C 37/08, C07C 39/04**

(21) Numéro de dépôt : **90403356.0**

(22) Date de dépôt : **27.11.90**

(54) **Procédé de production de phénol.**

(30) Priorité : **21.12.89 FR 8917162**

(43) Date de publication de la demande :
**26.06.91 Bulletin 91/26**

(45) Mention de la délivrance du brevet :
**29.12.93 Bulletin 93/52**

(84) Etats contractants désignés :
**BE CH DE ES FR GB IT LI NL SE**

(56) Documents cités :
**EP-A- 0 361 755
EP-A- 0 371 738
FR-A- 2 084 704
GB-A- 1 207 133
PATENT ABSTRACTS OF JAPAN vol. 11, no.
192 (C-429)(2639) 19 juin 1987, & JP-A-62 12729**

(56) Documents cités :
**PATENT ABSTRACTS OF JAPAN vol. 6, no.
110 (C-109)(988) 22 juin 1982, & JP-A-57 40423
Kirk-Othmer: "Encyclopedia of Chemical
Technology, 3. édition" 1982, Wiley Interscience, New York, US**

(73) Titulaire : **INSTITUT FRANCAIS DU PETROLE
4, avenue de Bois Préau
F-92502 Rueil-Malmaison (FR)**

(72) Inventeur : **Juguin, Bernard
46, avenue du Stade
F-92500 Rueil Malmaison (FR)**
Inventeur : **Boitiaux, Jean-Paul
4, avenue des Ursulines
F-78300 Poissy (FR)**
Inventeur : **Martino, Germain
Batiment Condé, 80, avenue F. Lefebvre
F-78300 Poissy (FR)**

Jouve, 18, rue Saint-Denis, 75001 PARIS

## Description

La présente invention concerne un procédé de production de phénol par la voie cumène.

Il existe de nombreux procédés de fabrication de phénol :

1) deux, les plus anciens, sont en voie de disparition : le premier opère par sulfonation du benzène suivie d'une fusion alcaline de l'acide sulfonique obtenu, le second par chloration catalytique suivie d'une hydrolyse basique du chlorobenzène obtenu ;

2) un autre procédé, également assez ancien, opère par oxydation du cumène issu de la réaction d'alkylation du benzène par le propène ;

3) deux autres, plus récents, ne représentent encore à ce jour qu'une faible partie de la production mondiale ; le premier met en oeuvre l'oxychloration du benzène, suivie d'une hydrolyse ; le second convertit le toluène par une oxydation catalytique au mécanisme complexe ;

4) enfin, le dernier en date met en oeuvre, dans une première étape, l'oxydation en phase liquide du cyclohexane , suivie, dans une seconde étape, d'une déshydrogénation catalytique des produits obtenus.

Le plus recherché, malgré sa relative ancienneté, reste le procédé mettant en oeuvre l'oxydation du cumène. Ce procédé comprend les deux phases suivantes :

- l'alkylation du benzène par le propène en cumène (isopropylbenzène) :
$$C_6H_6 + CH_3 - CH = CH_2 \Rightarrow C_6H_5 - CH - (CH_3)_2 \ (\Delta H = -26,6 \ kcal/mole)$$
- la transformation du cumène en phénol et acétone, qui comporte elle-même deux étapes principales :
  . l'oxydation du cumène en hydropéroxyde de cumyle :
$$C_6H_5\text{-}CH\text{-}(CH_3)_2 + O_2 \Rightarrow C_6H_5\text{-}COOH\text{-}(CH_3)_2 \ (\Delta H = -27,7 \ kcal/mole)$$
  . le clivage de l'hydropéroxyde de cumyle en phénol et acétone :
$$C_6H_5\text{-}COOH\text{-}(CH_3)_2 \Rightarrow C_6H_5OH + CH_3COCH_3 \ (\Delta H = -60,4 \ kcal/mole).$$

Il faut y ajouter généralement une troisième phase de purification avancée à cause de l'évolution des exigences des utilisateurs qui réclament un phénol de plus en plus pur.

Jusqu'à une période récente, cette voie au cumène tirait son avantage principal de la simplicité technique de l'opération, du rendement élevé en phénol par rapport au cumène et de la maîtrise que possèdent les détenteurs de procédés dans la manipulation de l'hydropéroxyde de cumyle.

De nos jours, le principal handicap de ce procédé est la coproduction obligatoire de 0,61 tonne d'acétone par tonne de phénol, car la demande en phénol croit beaucoup plus rapidement que celle en acétone.

Le procédé selon l'invention permet de pallier notamment à cet inconvénient économique. Il consiste à hydrogéner en partie ou en totalité l'acétone produite en alcool isopropylique, et à recycler au moins partiellement ce dernier à l'étape d'alkylation du benzène où, après déshydratation en propène, il sera à nouveau transformé en cumène. Cette invention permet donc d'équilibrer à la demande la production d'acétone entre 0 et 0,61 tonne par tonne de phénol produit.

Jusqu'ici, les techniques industrielles pour l'alkylation du benzène par le propène faisaient intervenir plusieurs types de catalyseurs, notamment le chlorure d'aluminium, l'acide phosphorique déposé sur un support solide, les silices-alumines. Ces divers catalyseurs encore très utilisés sont inadaptés pour la réaction d'alkylation du benzène en présence d'alcool isopropylique, car ils sont très sensibles à l'eau ; leur durée de vie en présence de l'eau formée par la déshydratation de l'alcool isopropylique serait donc assez réduite. Actuellement, seules certaines zéolithes sont bien adaptées au problème posé, car elles sont à la fois actives et sélectives et surtout stables en présence de vapeur d'eau.

Selon le procédé de l'invention, on fait d'abord réagir (première étape ou étape d'alkylation) du benzène avec une charge renfermant du propène et de l'alcool isopropylique (cet alcool isopropylique étant issu au moins en partie d'une quatrième étape ultérieure) au contact d'au moins un catalyseur à base d'une zéolithe Y désaluminée de rapport molaire $SiO_2/Al_2O_3$ compris entre 8 et 70, puis on fractionne le produit obtenu de manière à recueillir séparément une première fraction renfermant du benzène non-converti, une deuxième fraction renfermant du cumène et une troisième fraction renfermant au moins un polyisopropylbenzène (ou fraction polyisopropylbenzène).

Il peut être avantageux d'éliminer au moins la majeure partie de l'eau formée par la déshydratation de l'alcool isopropylique en propène lors de l'étape d'alkylation.

Dans une deuxième étape (étape de transalkylation), on fait réagir au moins une partie de ladite fraction polyisopropylbenzène avec du benzène, par exemple avec du benzène dont au moins une partie est constituée de benzène non-converti dans la première étape (c'est-à-dire de benzène non-converti provenant de ladite première fraction obtenue dans la première étape) ou par exemple avec du benzène dont au moins une partie n'est pas constituée de benzène non-reconverti dans la première étape, au contact d'au moins un catalyseur à base d'une zéolithe Y désaluminée de rapport molaire $SiO_2/Al_2O_3$ compris entre 8 et 70, ou bien, de manière préférée, au contact d'au moins un catalyseur à base d'une mordénite désaluminée de rapport atomique Si/Al global compris entre 20 et 60, et on recueille du cumène.

Le benzène excédentaire de la première étape qui n'est pas envoyé à la deuxième étape est avantageusement recyclé à la première étape, tandis que, après la deuxième étape, les polyisopropylbenzènes non convertis peuvent être au moins en partie recyclés dans cette même deuxième étape.

Ainsi, le cumène obtenu provient, d'une part, de la seconde fraction obtenue à la première étape et, d'autre part, de la réaction de la troisième fraction (fraction polyisopropylbenzène) obtenue à la première étape avec du benzène.

Dans une troisième étape, on transforme le cumène obtenu en un mélange de phénol et d'acétone, puis on fractionne ledit mélange de manière à recueillir séparément du phénol et de l'acétone.

Cette troisième étape comprend l'oxydation du cumène en hydropéroxyde de cumyle (par de l'oxygène par exemple), puis le clivage de cet hydropéroxyde de cumyle en phénol et acétone (par exemple sous l'action d'un acide), avant la purification et le fractionnement du mélange de phénol et d'acétone. Les phases d'oxydation, de clivage et de purification sont bien connues de l'homme du métier.

Dans une quatrième étape, on hydrogène au moins partiellement (de préférence en totalité) l'acétone obtenue à l'issue de la troisième étape en alcool isopropylique, de préférence en présence d'au moins un catalyseur à base de nickel déposé sur un support tel que la silice ou le kieselguhr, ledit alcool isopropylique étant ensuite au moins en partie recyclé vers la première étape d'alkylation du benzène.

La zéolithe Y désaluminée et la mordénite désaluminée sont chacune employées seules ou en mélange avec un liant ou une matrice généralement choisis dans le groupe formé par les argiles, les alumines, la silice, la magnésie, la zircone, l'oxyde de titane, l'oxyde de bore et toute combinaison d'au moins deux des composés précités comme la silice-alumine, la silice-magnésie etc... Toutes les méthodes connues d'agglomération et de mise en forme sont applicables, telles que, par exemple, l'extrusion, le pastillage, la coagulation en gouttes etc...

On utilise ainsi dans le procédé selon l'invention au moins un catalyseur à base de zéolithe Y désaluminée de rapport molaire $SiO_2/Al_2O_3$ compris entre 8 et 70, contenant généralement 1 à 100 %, de préférence 20 à 98 % et, par exemple, 40 à 98 % en poids de ladite zéolithe Y désaluminée et 0 à 99 %, de préférence 2 à 80 % et, par exemple, 2 à 60 % en poids d'une matrice, et éventuellement, au moins un catalyseur à base de mordénite désaluminée de rapport atomique Si/Al global compris entre 20 et 60, contenant généralement 1 à 100 %, de préférence 20 à 98 % et, par exemple, 40 à 98 % en poids de ladite mordénite désaluminée et 0 à 99 %, de préférence 2 à 80 % et, par exemple, 2 à 60 % en poids d'une matrice.

Les zéolithes Y désaluminées et leur préparation sont bien connues. On pourra par exemple se référer au brevet US 4738940.

La zéolithe Y utilisée dans la présente invention est une zéolithe acide HY caractérisée par différentes spécifications : un rapport molaire $SiO_2/Al_2O_3$ compris entre 8 et 70 et, de manière préférée, entre environ 12 et 40 ; une teneur en sodium inférieure à 0,25 % poids déterminée sur la zéolithe calcinée à 1100°C ; un paramètre cristallin a de la maille élémentaire compris entre $24,55 \times 10^{-10}$ et $24,24 \times 10^{-10}$ m et, de manière préférée, entre $24,39 \times 10^{-10}$ et $24,26 \times 10^{-10}$ m ; une capacité $C_{Na}$ de reprise en ions sodium, exprimée en gramme de Na par 100 grammes de zéolithe modifiée neutralisée puis calcinée, supérieure à environ 0,85 ; une surface spécifique déterminée par la méthode B.E.T. supérieure à environ 400 m²/g et, de préférence, supérieure à 550 m²/g ; une capacité d'adsorption de vapeur d'eau à 25°C, pour une pression partielle de 2,6 torrs, supérieure à environ 6 % ; une répartition poreuse comprenant entre 1 et 20 % et, de préférence, entre 3 et 15 % du volume poreux contenu dans des pores de diamètre situé entre $20 \times 10^{-10}$ et $80 \times 10^{-10}$ m, le reste du volume poreux étant contenu dans les pores de diamètre inférieur à $20 \times 10^{-10}$ m.

Cette zéolithe Y est par exemple fabriquée, généralement à partir d'une zéolithe Y-Na, par une combinaison appropriée de deux traitements de base : (a) un traitement hydrothermique qui associe température et pression partielle de vapeur d'eau, et (b) un traitement acide par, de préférence, un acide minéral fort et concentré.

Généralement la zéolithe Y-Na à partir de laquelle on prépare la zéolithe Y utilisée dans l'invention possède un rapport molaire $SiO_2/Al_2O_3$ compris entre environ 4 et 6 ; il conviendra au préalable d'en abaisser la teneur pondérale en sodium à moins de 3 % et, de préférence, à moins de 2,5 % ; la zéolithe Y-Na possède en outre généralement une surface spécifique comprise entre environ 750 et 950 m²/g.

Les mordénites désaluminées et leur préparation sont également bien connues. On pourra par exemple se référer aux brevets européens EP-0084746, 0097552 et 0196965 et à la demande de brevet français de numéro d'enregistrement national 87/12932. La méthode de désalumination des brevets ou de la demande précités consiste à soumettre la forme H de la mordénite ou un précurseur de forme H (par exemple la forme $NH_4$) à une série de chauffages en atmosphère de vapeur d'eau et de traitements acides. Toutefois, par différence avec les traitements desdits documents précités, on limitera ici le taux de désalumination à un rapport atomique global Si/Al entre 20 et 60. On opère de préférence comme suit :

- dans une première étape, on élimine les cations non décomposables, généralement Na$^+$, présents dans la mordénite de départ. Pour ce faire, on pourra procéder à un ou plusieurs échanges dans des solutions diluées d'acides comme HCl ou dans des solutions de NH$_4$$^+$, éventuellement suivis d'un ou plusieurs lavages (à l'eau par exemple). Le point important est que, à l'issue de cette première étape que l'on peut qualifier de décationisation, la quasi-totalité des cations alcalins soit éliminée (teneur en Na par exemple comprise entre 150 et 2000 ppm en poids et, de préférence, entre 300 et 1200 ppm en poids) et le solide obtenu soit une forme H ou un précurseur de forme H (exemple NH$_4$$^+$) non désaluminée substantiellement (taux de désalumination généralement inférieur à 10 % et, de préférence, à 5 %). De manière préférée, on choisira, comme précurseur de forme H, la forme NH$_4$$^+$,

- dans une deuxième étape, la forme H ou le précurseur de forme H est soumis à un traitement sous vapeur d'eau à une température supérieure à 450°C, par exemple comprise entre 450 et 650°C et, de préférence, entre 550 et 600°C. La teneur en eau (en volume) de l'atmosphère de calcination sera avantageusement supérieure à 20 % et, de manière préférée, à 40 %,

- l'attaque acide constitue la troisième étape de la préparation des catalyseurs. Pour des rapports atomiques Si/Al de charpente (du solide après calcination) jusqu'à 50 environ, on utilisera de préférence des concentrations de solutions acides (HCl, H$_2$SO$_4$, HNO$_3$, etc...) comprises entre 0,5 et 5N et, de préférence, entre 1 et 4N. Pour des rapports atomiques Si/Al de charpente supérieurs, on utilisera des concentrations de solutions acides comprises entre 5 et 20 N et, de préférence, entre 7 et 12N (les rapports atomiques Si/Al de charpente peuvent être déterminés par spectroscopie infra-rouge pour des rapports compris entre 10 et 50 et par RMN du $^{29}$Si pour des rapports supérieurs). Par ailleurs, pour atteindre des rapports atomiques Si/Al élevés, c'est-à-dire supérieurs à environ 50, on pourra recourir avantageusement à plusieurs cycles calcination sous vapeur d'eau-attaque acide.

Les solides ainsi préparés ont avantageusement des rapports atomiques Si/Al globaux de 20 à 60 ; ils ont un volume de maille élémentaire situé entre 2,755 et 2,730 nm$^3$ (1 nm = 10$^{-9}$ m) et, de préférence, entre 2,745 et 2,735 nm$^3$ ; ils ont de préférence une force acide suffisante pour que les Al-OH structuraux intéragissent avec une base faible comme l'éthylène (mesure infra-rouge à 77 K) ou un composé à caractère faiblement acide comme H$_2$S (mesure infra-rouge à 25°C). Ces solides doivent, en outre, être de préférence exempts d'espèces cationiques extra-réseau que l'on peut détecter par un signal fin (largeur à mi-hauteur inférieure à 5 ppm et, de préférence, inférieure à 2 ppm) situé à 0 ppm (référence Al(H$_2$O)$_6$ $^{3+}$) sur un spectre de RMN de $^{27}$Al, mesuré avec la technique de rotation de l'angle magique.

La réaction dite d'alkylation (première étape du procédé selon l'invention) est habituellement effectuée en phase liquide, en phase supercritique ou en phase gazeuse, en présence d'au moins un catalyseur à base de la zéolithe Y désaluminée définie plus haut, disposé en lit fixe, à une température d'environ 100 à 350°C (de préférence d'environ 180 à 300°C), sous une pression de 1 à 10 MPa (de préférence de 2 à 7 MPa), avec un débit d'hydrocarbures liquides (vitesse spatiale) d'environ 0,5 à 20 volumes par volume de catalyseur et par heure, et avec un rapport molaire benzène/(propène + alcool isopropylique) compris entre 1 et 20 (de préférence entre 5 et 12).

La réaction dite de transalkylation des polyisopropylbenzènes (deuxième étape du procédé selon l'invention) formés au cours de l'étape d'alkylation est habituellement effectuée en présence d'au moins un catalyseur à base de la zéolithe Y désaluminée définie plus haut ou, de préférence, à base de la mordénite désaluminée répondant aux caractéristiques générales décrites plus haut, disposé en lit fixe, à une température d'environ 250 à 500°C (de préférence d'environ 300 à 420°C), sous une pression de 2 à 10 MPa (de préférence de 2,5 à 7 MPa), avec un débit d'hydrocarbures liquides (vitesse spatiale) d'environ 0,3 à 5 volumes par volume de catalyseur et par heure, et avec un rapport molaire benzène/polyisopropylbenzènes de 2 à 50 (de préférence de 5 à 20).

La réaction d'hydrogénation de l'acétone en alcool isopropylique (quatrième étape du procédé selon l'invention) est habituellement effectuée en présence d'au moins un catalyseur à base de nickel déposé sur un support tel que la silice ou le kieselguhr, contenant généralement 3 à 70 %, de préférence 8 à 60 % et, par exemple, 12 à 25 % en poids de nickel (ce catalyseur peut être utilisé sous forme de billes, de pastilles ou d'extrudés), disposé en lit fixe, à une température d'environ 40 à 200°C (de préférence d'environ 60 à 160°C), sous une pression de 1,5 à 10 MPa (de préférence de 2 à 6 MPa), avec un débit horaire d'hydrocarbures liquides (acétone + éventuellement alcool isopropylique recyclés) d'environ 1 à 5 fois le poids de catalyseur, avec un rapport molaire hydrogène/acétone compris entre 1 et 20 (de préférence entre 3 et 8). La réaction d'hydrogénation de l'acétone étant exothermique, on limite généralement l'augmentation de température entre l'entrée et la sortie du réacteur d'hydrogénation à une valeur de 30 à 70°C environ pour ne pas risquer un emballement de la réaction ; pour ce faire, on recycle en général au moins une partie de l'alcool isopropylique formé à l'entrée du réacteur d'hydrogénation, avec un rapport molaire alcool isopropylique/acétone à l'entrée dudit réacteur compris entre 2 et 20 (de préférence entre 4 et 15).

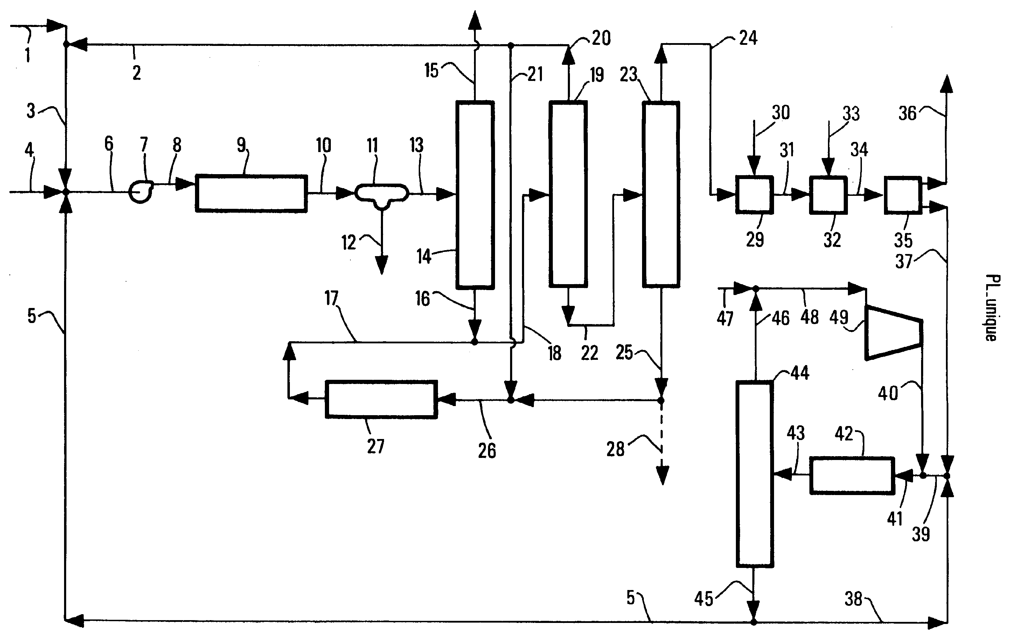

La figure unique illustre un mode particulier de réalisation de l'invention : du benzène frais (1) est mélangé avec du benzène recyclé (2) issu de la tête d'une colonne à distiller (19) ; le mélange résultant (3) est à son tour mélangé avec du propène frais (4) et avec de l'alcool isopropylique (5) issu du fond d'une colonne à distiller (44). Le mélange obtenu (6) est alors envoyé, à l'aide d'une pompe (7), vers le réacteur d'alkylation (9) par la conduite (8), après passage dans un four de réchauffage non représenté sur la figure. A la sortie du réacteur d'alkylation (9), l'effluent est introduit par la conduite (10) dans un ballon décanteur (11). En fond du ballon décanteur (11), on soutire par la conduite (12) la majeure partie de l'eau formée à la suite de la déshydratation de l'alcool isopropylique en propène. La fraction hydrocarburée surnageante est alors envoyée, par la conduite (13), vers une première colonne à distiller (14) jouant le rôle de dépropaniseur ; au sommet de cette première colonne à distiller (14), on recueille, par la conduite (15), un mélange formé de vapeur d'eau, de propane et de propène non transformés au cours de la réaction d'alkylation. En fond de cette colonne à distiller (14), on soutire, par la conduite (16), un mélange de benzène non-converti et d'alkylbenzènes (cumène, polyisopropylbenzène), qui est ensuite mélangé avec des hydrocarbures issus, par la conduite (17), du réacteur de transalkylation (27). Le mélange résultant est alors envoyé, par la conduite (18), vers une deuxième colonne à distiller (19). Au sommet de cette deuxième colonne à distiller (19), on recueille, par la conduite (20), le benzène en excès non transformé dans le réacteur d'alkylation ; ce benzène est en partie recyclé, par la conduite (2), vers le réacteur d'alkylation (9), l'autre partie étant envoyée, par la conduite (21), vers le réacteur de transalkylation des polyisopropylbenzènes (27). En fond de cette deuxième colonne à distiller (19), on soutire, par la conduite (22), un mélange de cumène et de polyisopropylbenzènes, qui est envoyé vers une troisième colonne à distiller (23). Au sommet de cette troisième colonne à distiller (23), on recueille du cumène pur, qui est envoyé, par la conduite (24), vers le réacteur d'oxydation du cumène (29). Au fond de cette troisième colonne à distiller (23), on soutire, par la conduite (25), un produit constitué de diisopropylbenzènes et de triisopropylbenzènes qui, après mélange avec le benzène recyclé par la conduite (21), est envoyé, par la conduite (26),   vers   le réacteur de transalkylation des polyisopropylbenzènes (27). Si nécessaire, une partie des polyisopropylbenzènes peut éventuellement être purgée du circuit par la conduite (28). Le cumène issu du sommet de la troisième colonne à distiller (22) par la conduite (24) entre alors dans le réacteur d'oxydation (29), où il est transformé en hydropéroxyde de cumyle (apport d'oxygène par la conduite (30)), qui est lui-même envoyé, par la conduite (31), dans la section de clivage (32), pour y être transformé en un mélange de phénol et d'acétone (apport d'acide par la conduite (33)). Ce mélange est ensuite envoyé, par la conduite (34), dans une section (35) pour y être purifié et séparé par fractionnement. A la sortie de la section (35) de purification et de fractionnement, on recueille, d'une part, du phénol pur qui est envoyé, par la conduite (36), au stockage, et, d'autre part, de l'acétone pure (conduite (37)). Cette acétone pure est ensuite mélangée avec de l'alcool isopropylique (utilisé comme diluant) issu, par les conduites (45) puis (38), du fond d'une quatrième colonne à distiller (44). Le mélange résultant (39) est alors mélangé à de l'hydrogène sous pression en provenance, par la conduite (40), d'un compresseur (49). L'hydrogène entrant dans le compresseur (49), par la conduite (48), est constitué, d'une part, d'hydrogène de recyclage issu, par la conduite (46), du sommet de la quatrième colonne à distiller (44), et, d'autre part, d'hydrogène frais arrivant par la conduite (47). Le mélange acétone-alcool isopropylique-hydrogène est introduit, par la conduite (41), dans le réacteur d'hydrogénation (42). L'effluent du réacteur d'hydrogénation (42) est alors envoyé, par la conduite (43), dans la quatrième colonne à distiller (44). Au sommet de cette quatrième colonne (44), on recueille, par la conduite (46), l'hydrogène non transformé. En fond de cette quatrième colonne à distiller (44), on soutire, par la conduite (45), de l'alcool isopropylique ; une partie de cet alcool est recyclé, par la conduite (38), vers le réacteur d'hydrogénation (42) pour servir de diluant, l'autre partie étant renvoyée, par la conduite (5), vers le réacteur d'alkylation du benzène (9).

Les exemples suivants, non limitatifs, illustrent l'invention sans toutefois en limiter la portée.

EXEMPLE 1 : Préparation d'un catalyseur A.

On utilise comme matière première une zéolithe NaY de formule $Na\ AlO_2\ (SiO_2)_{2,5}$.
Cette zéolithe présente les caractéristiques suivantes :
- rapport molaire $SiO_2/Al_2O_3$        : 5
- paramètre cristallin a        : 24,69 x $10^{-10}$ m
- capacité d'adsorption de vapeur d'eau à 25°C (à P/Po = 0,1)        : 26%
- surface spécifique        : 880 $m^2$/g.

Elle est soumise à cinq échanges consécutifs dans des solutions de nitrate d'ammonium de concentration 2M, à une température de 95°C, pendant un temps de 1,5 heure, et avec un rapport volume de solution sur poids de zéolithe égal à 8 $cm^3$/g. Le taux de sodium de la zéolithe $NaNH_4Y$ obtenu est 0,95%. Ce produit est ensuite introduit rapidement dans un four préchauffé à 770°C et laissé pendant 4 heures en atmosphère statique (traitement stabilisant). La zéolithe est ensuite soumise à un traitement acide dans les conditions suivantes : le rapport entre le volume de solution d'acide nitrique 2N et le poids de solide est de 6 cm³/g, la température est de 95°C et la durée du traitement de 3 heures. Ensuite un autre traitement dans les mêmes conditions est effectué, mais avec une solution d'acide nitrique 0,3N.

La zéolithe obtenue possède une teneur pondérale en sodium de 0,2 %, un rapport molaire $SiO_2/Al_2O_3$ de 18, un paramètre cristallin a de la maille élémentaire de $24,32 \times 10^{-10}$ m, une surface spécifique de 805 m²/g, une capacité de reprise en eau de 13,7 % (à P/Po = 0,1), une capacité de reprise en ions sodium de 2 et 9 % de son volume poreux est contenu dans des pores de diamètre situé entre $20 \times 10^{-10}$ et $80 \times 10^{-10}$ m, le reste de son volume poreux étant contenu dans des pores de diamètre inférieur à $20 \times 10^{-10}$ m.

Cette zéolithe est ensuite mise en forme par extrusion avec de l'alumine. Les extrudés obtenus sont ensuite séchés puis calcinés vers 500°C environ. On obtient alors un catalyseur A à base de ladite zéolithe, contenant 80 % en poids de ladite zéolithe et 20 % en poids d'alumine.

EXEMPLE 2 : Préparation d'un catalyseur B.

La matière première utilisée pour préparer ce catalyseur est une mordénite petits pores de la Société Chimique de la Grande Paroisse, référencée Alite 150 ; sa formule chimique à l'état anhydre est Na, $AlO_2(SiO_2)_{5,5}$ et sa teneur pondérale en sodium de 5,3 %. 500 grammes de cette poudre sont plongés dans une solution 2 M de nitrate d'ammonium, et la suspension est portée à 95°C pendant 2 heures. Le volume de la solution de nitrate d'ammonium engagée est égal à 4 fois le poids de mordénite sèche (V/P = 4 g/cm³). Cette opération d'échange cationique est recommencée 3 fois. Après le 3ème échange, le produit est lavé à l'eau à 20°C pendant 20 minutes, avec un rapport V/P égal à 4 g/cm³. La teneur en sodium exprimée en pourcentage en poids par rapport à la mordénite sèche n'est plus que de 0,1 %. Le produit est ensuite filtré, et soumis à une calcination sous vapeur d'eau en atmosphère confinée ("self-steaming") à 560°C pendant 2 heures. La teneur en eau (en volume) de l'atmosphère de calcination est de l'ordre de 90 %. La cristallinité de ce solide, après cette étape de calcination, est supérieure ou égale à 90 % ; son rapport atomique Si/Al de charpente est égal à 49.

On procède ensuite sur ce solide à une attaque acide à l'aide d'une solution d'acide nitrique 3,5N. Au cours de l'attaque acide, le solide est ainsi porté à reflux dans la solution d'acide nitrique pendant 2 heures, avec un rapport V/P égal à 8 g/cm³. Le produit est ensuite filtré, puis lavé abondamment à l'eau distillée.

La mordénite obtenue possède un rapport atomique Si/Al global de 49 ; son volume de maille élémentaire est égal à 2,74 nm³.

Elle est ensuite mise en forme par malaxage avec de l'alumine, puis par passage au travers d'une filière. Les extrudés obtenus, de diamètre 1,2 mm, sont ensuite séchés, puis calcinés entre 150 et 500°C par palier d'une heure environ. On obtient alors un catalyseur B à base de ladite mordénite, contenant 80 % en poids de ladite mordénite et 20 % en poids d'alumine.

EXEMPLE 3

Une charge est envoyée dans un réacteur d'alkylatiuon contenant le catalyseur A.
Les conditions opératoires dans le réacteur d'alkylation sont les suivantes :
- température : 240°C
- pression : 4 MPa
- débit horaire de benzène égal à 2 fois le volume du catalyseur
- rapport molaire benzène/propène : 8,4.
La charge a la composition pondérale suivante :
- propane : 0,31 %
- propène : 6,00 %
- benzène : 93,69 %
A la sortie du réacteur d'alkylation, le produit obtenu a la composition pondérale suivante :
- propane : 0,31 %
- propène : -
- benzène : 82,99 %
- cumène : 15,83 %
- diisopropylbenzènes : 0,77 %
- triisopropylbenzènes : 0,10 %
Le produit obtenu est ensuite fractionné par distillation, et deux fractions sont recueillies :
- une fraction point initial - 160° contenant le benzène non converti et le cumène, le cumène étant ensuite séparé de ladite fraction,
- une fraction de point d'ébullition supérieur à 160°C contenant les polyisopropylbenzènes.

Cette fraction de point d'ébullition supérieur à 160°C subit ensuite un traitement de transalkylation en présence du catalyseur B préparé dans l'exemple 2.

Les conditions opératoires dans le réacteur de transalkylation sont les suivantes :
- température : 350°C
- pression : 4 MPa
- débit horaire de (benzène + polyisopropylbenzènes) égal à 1 fois le volume du catalyseur
- rapport molaire benzène/polyisopropylbenzènes : 15.

La charge a donc la composition pondérale suivante :

- benzène : 6,13
- diisopropylbenzènes : 0,77
- triisopropylbenzènes : 0,10
                          ———
                           7

A la sortie du réacteur de transalkylation, le produit obtenu a la composition pondérale suivante :

- benzène : 5,79
- cumène : 1,10
- diisopropylbenzènes : 0,08
- triisopropylbenzènes : 0,03
                          ———
                           7

Les résultats d'ensemble combinant les deux étapes d'alkylation d'une part et de transalkylation d'autre part sont les suivants :
- composition pondérale du produit obtenu :

propane : 0,31 %
propène : -
benzène : 82,65 %
cumène : 16,93 %
diisopropylbenzènes : 0,08 %
triisopropylbenzènes : 0,03 %
- taux de transformation du benzène : 11,8 %
- taux de transformation du propène : 100 %
- sélectivité en cumène par rapport au propène transformé : 98,8 %
- sélectivité en cumène par rapport au benzène transformé : 99,7 %

Conformément aux données publiées dans la littérature scientifique et commerciale (Procédés de Pétrochimie, Société des éditions TECHNIP, 1971, page 356), 16,93 kg de cumène conduiraient, après oxydation, clivage acide, purifications et fractionnements, à la production conjointe de 12,05 kg de phénol pur et 7,38 kg d'acétone, ce qui correspond à des consommations spécifiques, par tonne de phénol pur produit, de 0,497 tonne de propène et de 0,916 tonne de benzène.

EXEMPLE 4

Un catalyseur constitué par des extrudés contenant 20 % en poids de nickel et 80 % en poids de silice, est utilisé pour la réaction d'hydrogénation de l'acétone en alcool isopropylique.

Les conditions opératoires dans le réacteur d'hydrogénation sont les suivantes :
- température d'entrée dans le réacteur : 80°
- température de sortie du réacteur : 130°
- pression opératoire : 4 MPa
- rapport molaire alcool isopropylique/acétone : 6,6
- débit horaire de charge (acétone + alcool isopropylique) égal à 2 fois le poids du catalyseur

- rapport molaire hydrogène/acétone        : 5.

La charge a donc la composition pondérale suivante :

- hydrogène        : 2,19 %
- alcool isopropylique        : 85,09 %
- acétone        : 12,72 %

A la sortie du réacteur d'hydrogénation, le produit obtenu a la composition pondérale suivante :

- hydrogène        : 1,75 %
- alcool isopropylique        : 98,25 %

La réaction est donc quantitative.

Dans le cas de l'exemple 3, 7,38 kg d'acétone conduisent donc à la production de 7,63 kg d'alcool isopropylique.

## EXEMPLE 5

Le catalyseur A préparé dans l'exemple 1 est utilisé pour la réaction d'alkylation du benzène par l'alcool isopropylique.

Les conditions opératoires dans le réacteur d'alkylation sont les suivantes :

- températures        : 240°C
- pression        : 4 MPa
- débit horaire de charge (benzène + alcool isopropylique) égal à 2 fois le volume du catalyseur
- rapport molaire benzène/alcool isopropylique        : 8,8.

La charge à l'entrée du réacteur d'alkylation a donc la composition pondérale suivante :

- benzène        : 92 %
- alcool isopropylique        : 8 %.

A la sortie du réacteur d'alkylation, le produit obtenu a la composition pondérale suivante :

- eau        : 2,40 %
- propène        : 0,05 %
- benzène        : 82,64 %
- cumène        : 13,14 %
- diisopropylbenzènes        : 1,45 %
- triisopropylbenzènes        : 0,32 %

Après réaction, l'eau formée est séparée par décantation, puis la phase hydrocarburée est fractionée par distillation, et deux fractions sont recueillies :

- une fraction point initial -160°C contenant le benzène non converti et le cumène, le cumène étant ensuite séparé de ladite fraction,
- une fraction de point d'ébullition supérieur à 160°C contenant les polyisopropylbenzènes.

Cette fraction de point d'ébullition supérieur à 160°C est ensuite soumise à un traitement de transalkylation en présence du catalyseur B préparé dans l'exemple 2.

Les conditions opératoires dans le réacteur de transalkylation sont les suivantes :

- température        : 350°C
- pression        : 4 MPa
- débit horaire de benzène + polyisopropylbenzènes égal à 1 fois le volume du catalyseur,
- rapport molaire benzène/polyisopropylbenzènes        : 15.

La charge a donc la composition pondérale suivante :

```
- benzène              : 12,31
- diisopropylbenzènes  :  1,45
- triisopropylbenzènes :  0,32
                          ─────
                         14,08
```

A la sortie du réacteur de transalkylation, le produit obtenu a la composition pondérale suivante :

```
- benzène             :  11,65
- cumène              :   2,18
- diisopropylbenzènes :   0,15
- triisopropylbenzènes :  0,10
                          14,08
```

Les résultats d'ensemble combinant les deux étapes d'alkylation d'une part et de transalkylation d'autre part sont les suivants :

- composition pondérale du produit :

eau                      : 2,40 %
propène                  : 0,05 %
benzène                  : 81,98 %
cumène                   : 15,32 %
diisopropylbenzènes      : 0,15 %
triisopropylbenzènes     : 0,10 %

- taux de transformation du benzène          : 10,9 %
- taux de transformation de l'alcool isopropylique          : 100 %
- sélectivité en cumène par rapport à l'alcool isopropylique transformé          : 95,8 %
- sélectivité en cumène par rapport au benzène transformé          : 99,4 %

Après oxydation, clivage acide, purifications et fractionnements, 15,32 kgs de cumène conduiraient à la production conjointe de 10,91 kg de phénol pur et 6,68 kg d'acétone, ce qui correspond à des consommation spécifiques, par tonne de phénol pur produit, de 0,733 tonne d'alcool isopropylique et de 0,918 tonne de benzène.

## EXEMPLE 6

Conformément à l'invention, le catalyseur A préparé dans l'exemple 1 est utilisé pour la réaction d'alkylation du benzène par un mélange de propène et d'alcool isopropylique. Le rapport pondéral alcool isopropylique/propène est conforme aux résultats obtenus dans les exemples 3 et 4.

Les conditions opératoires dans le réacteur d'alkylation sont les suivantes :

- température          : 240°C
- pression          : 4 MPa
- débit horaire de charge (benzène + propène + alcool isopropylique) égal à 2 fois le volume du catalyseur
- rapport molaire benzène/propène + alcool isopropylique          : 8,4.

La charge à l'entrée du réacteur d'alkylation a donc la composition pondérale suivante :

- propane          : 0,15 %
- propène          : 3,13 %
- alcool isopropylique          : 3,99 %
- benzène          : 92,73 %.

A la sortie du réacteur d'alkylation, le produit obtenu a la composition pondérale suivante :

- eau          : 1,20 %
- propane          : 0,15 %
- propène          : 0,03 %
- benzène          : 82,49 %
- cumène          : 14,80 %
- diisopropylbenzènes          : 1,12 %
- triisopropylbenzènes          : 0,21 %

Après réaction, l'eau formée est séparée par décantation, puis la phase hydrocarburée est fractionnée par distillation, et deux fractions sont recueillies :

- une fraction point initial -160°C contenant le benzène non converti et le cumène, le cumène étant ensuite séparée de ladite fraction,
- une fraction de point d'ébullition supérieur à 160°C contenant les polyisopropylbenzènes.

Cette fraction de point d'ébullition supérieur à 160°C est ensuite soumise à un traitement de transalkylation en présence du catalyseur B préparé dans l'exemple 2.

Les conditions opératoires dans le réacteur de transalkylation sont les suivantes :
- température         : 350°C
- pression            : 4 MPa
- débit horaire de benzène + polyisopropylbenzènes égal à 1 fois le volume du catalyseur
- rapport molaire benzène/polyisopropylbenzènes          : 15.
La charge a donc la composition pondérale suivante :

```
- benzène              : 9,29
- diisopropylbenzènes  : 1,12
- triisopropylbenzènes : 0,21
                         10,62
```

A la sortie du réacteur de transalkylation, le produit obtenu a la composition pondérale suivante :

```
- benzène              : 8,78
- cumène               : 1,67
- diisopropylbenzènes  : 0,11
- triisopropylbenzènes : 0,06
                         10,62
```

Les résultats d'ensemble combinant les deux étapes d'alkylation et de transalkylation sont les suivants :
- composition pondérale du produit :

eau                       : 1,20 %
propane                   : 0,15 %
propène                   : 0,03 %
benzène                   : 81,98 %
cumène                    : 16,47 %
diisopropylbenzènes       : 0,11 %
triisopropylbenzènes      : 0,06 %
- taux de transformation du benzène          : 11,6 %
- taux de transformation du propène          : 99 %
- taux de transformation de l'alcool isopropylique          : 100 %
- sélectivité en cumène par rapport à la somme propène + alcool isopropylique transformés          : 97,8 %
- sélectivité en cumène par rapport au benzène transformé          : 99,6 %

Après oxydation du cumène, clivage acide de l'hydropéroxyde de cumyle, purifications et fractionnements, 16,47 kg de cumène conduisent à la production de 11,73 kg de phénol pur, ce qui correspond à des consommations spécifiques, par tonne de phénol pur produit, de 0,267 tonne de propène et de 0,916 tonne de benzène.

Si l'on compare les résultats obtenus dans les exemples 3 et 6 on s'aperçoit du grand avantage de travailler selon l'invention, car :

1) il est possible d'obtenir du phénol pur sans production conjointe d'acétone,

2) pour une même consommation spécifique de benzène, la consommation spécifique de propène est réduite de 46 %.

Selon les fluctuations de marché, il est possible également d'équilibrer à la demande la production conjointe d'acétone, en jouant sur le taux de recyclage de ce co-produit vers l'unité d'hydrogénation. L'invention apporte donc notamment une grande flexibilité opératoire dans ce type de procédé.

**Revendications**

1.  Procédé de production de phénol caractérisé en ce qu'il comprend les étapes successives suivantes :
    1) on fait réagir du benzène avec une charge renfermant du propène et de l'alcool isopropylique au contact d'au moins un catalyseur à base d'une zéolithe Y désaluminée de rapport molaire $SiO_2/Al_2O_3$ compris entre 8 et 70, puis on fractionne le produit obtenu de manière à recueillir séparément une première fraction renfermant du benzène non-converti, une deuxième fraction renfermant du cumène et une troisième fraction renfermant au moins un polyisopropylbenzène,

2) on fait réagir au moins une partie de ladite troisième fraction avec du benzène au contact d'au moins un catalyseur à base d'une zéolithe Y désaluminée de rapport molaire $SiO_2/Al_2O_3$ compris entre 8 et 70, et on recueille du cumène,

3) on transforme le cumène provenant des étapes 1) et 2) en un mélange de phénol et d'acétone, puis on fractionne ledit mélange de manière à recueillir séparément du phénol et de l'acétone,

4) on hydrogène au moins partiellement l'acétone obtenue à l'issue de l'étape 3) en alcool isopropylique que l'on recycle ensuite au moins en partie vers l'étape 1).

**2.** Procédé de production de phénol caractérisé en ce qu'il comprend les étapes successives suivantes :

1) on fait réagir du benzène avec une charge renfermant du propène et de l'alcool isopropylique au contact d'au moins un catalyseur à base d'une zéolithe Y désaluminée de rapport molaire $SiO_2/Al_2O_3$ compris entre 8 et 70, puis on fractionne le produit obtenu de manière à recueillir séparément une première fraction renfermant du benzène non-converti, une deuxième fraction renfermant du cumène et une troisième fraction renfermant au moins un polyisopropylbenzène,

2) on fait réagir au moins une partie de ladite troisième fraction avec du benzène au contact d'au moins un catalyseur à base d'une mordénite désaluminée de rapport atomique Si/Al global compris entre 20 et 60, et on recueille du cumène,

3) on transforme le cumène provenant des étapes 1) et 2) en un mélange de phénol et d'acétone, puis on fractionne ledit mélange de manière à recueillir séparément du phénol et de l'acétone,

4) on hydrogène au moins partiellement l'acétone obtenue à l'issue de l'étape 3) en alcool isopropylique que l'on recycle ensuite au moins en partie vers l'étape 1).

**3.** Procédé selon l'une des revendications 1 et 2 dans lequel au moins une partie du benzène utilisé dans l'étape 2) est constituée de benzène non converti provenant de ladite première fraction obtenue à l'issue de l'étape 1).

**4.** Procédé selon l'une des revendications 1 à 3 dans lequel les polyisopropylbenzènes non-convertis lors de l'étape 2) sont au moins en partie recyclés dans ladite étape 2).

**5.** Procédé selon l'une des revendications 1 à 4 dans lequel la transformation du cumène en un mélange de phénol et d'acétone lors de l'étape 3) comprend l'oxydation du cumène en hydropéroxyde de cumyle, puis le clivage dudit hydropéroxyde de cumyle en un mélange de phénol et d'acétone.

**6.** Procédé selon l'une des revendications 1 à 5 dans lequel l'étape 4) d'hydrogénation s'effectue en présence d'au moins un catalyseur à base de nickel déposé sur un support.

**7.** Procédé selon l'une des revendications 1 à 6 dans lequel on élimine, à l'issue de l'étape 1), au moins la majeure partie de l'eau formée par la déshydratation de l'alcool isopropylique en propène lors de l'étape 1).

**8.** Procédé selon l'une des revendications 1 à 7 dans lequel chaque catalyseur est disposé en lit fixe.

**9.** Procédé de production de phénol pur selon l'une des revendications 1 à 8 sans production conjointe d'acétone.

**Patentansprüche**

**1.** Verfahren zur Herstellung von Phenol, dadurch gekennzeichnet, daß es die folgenden aufeinanderfolgenden Stufen umfaßt:

1) im Kontakt mit mindestens einem Katalysator auf Basis eines desaluminierten Y-Zeoliths mit einem $SiO_2/Al_2O_3$-Molverhältnis zwischen 8 und 70 läßt man Benzol mit einer Beschickung reagieren, die Propen und Isopropylalkohol enthält, dann fraktioniert man das erhaltene Produkt in der Weise, daß man getrennt voneinander erhält eine nicht-umgewandeltes Benzol enthaltende erste Fraktion, eine Cumol enthaltende zweite Fraktion und eine mindestens ein Polyisopropylbenzol enthaltende dritte Fraktion,

2) im Kontakt mit mindestens einem Katalysator auf Basis eines desaluminierten Y-Zeoliths mit einem $SiO_2/Al_2O_3$-Molverhältnis zwischen 8 und 70 läßt man mindestens einen Teil der genannten dritten Fraktion mit Benzol reagieren und man gewinnt Cumol,

3) man wandelt das aus den Stufen (1) und (2) stammende Cumol in ein Gemisch aus Phenol und Aceton um, dann fraktioniert man das genannte Gemisch in der Weise, daß man getrennt Phenol und Aceton erhält,

4) man hydriert das am Ende der Stufe (3) erhaltene Aceton mindestens teilweise zu Isopropylalkohol, den man anschließend mindestens teilweise im Kreislauf in die Stufe (1) zurückführt.

2. Verfahren zur Herstellung von Phenol, dadurch gekennzeichnet, daß es die folgenden aufeinanderfolgenden Stufen umfaßt:

1) im Kontakt mit mindestens einem Katalysator auf Basis eines desaluminierten Y-Zeoliths mit einem $SiO_2/Al_2O_3$-Molverhältnis zwischen 8 und 70 läßt man Benzol mit einer Beschickung reagieren, die Propen und Isopropylalkohol enthält, dann fraktioniert man das erhaltene Produkt in der Weise, daß man getrennt erhält eine nicht-umgewandeltes Benzol enthaltende erste Fraktion, eine Cumol enthaltende zweite Fraktion und eine mindestens ein Polyisorpopylbenzol enthaltende dritte Fraktion,

2) im Kontakt mit mindestens einem Katalysator auf Basis eines desaluminierten Mordenits mit einem Si/Al-Gesamtverhältnis zwischen 20 und 60 läßt man mindestens einen Teil der genannten dritten Fraktion mit Benzol reagieren und gewinnt Cumol,

3) man wandelt das aus den Stufen (1) und (2) stammende Cumol in ein Gemisch aus Phenol und Aceton um, dann fraktioniert man das genannte Gemisch in der Weise, daß man getrennt Phenol und Aceton erhält, und

4) man hydriert das am Ende der Stufe (3) erhaltene Aceton mindestens teilweise zu Isopropylalkohol, den man anschließend mindestens teilweise im Kreislauf in die Stufe (1) zurückführt.

3. Verfahren nach einem der Ansprüche 1 und 2, in dem mindestens ein Teil des in der Stufe (2) verwendeten Benzols aus nicht-umgewandeltem Benzol besteht, das aus der genannten ersten Fraktion stammt, die am Ende der Stufe (1) erhalten wird.

4. Verfahren nach einem der Ansprüche 1 bis 3, in dem die in der Stufe (2) nicht-umgewandelten Polyisopropylbenzole mindestens teilweise im Kreislauf in die genannte Stufe (2) zurückgeführt werden.

5. Verfahren nach einem der Ansprüche 1 bis 4, in dem die Umwandlung von Cumol in ein Gemisch aus Phenol und Aceton in der Stufe (3) umfaßt die Oxidation von Cumol zu Cumylhydroperoxid und die anschließende Spaltung des genannten Cumylhydroperoxids zu einem Gemisch aus Phenol und Aceton.

6. Verfahren nach einem der Ansprüche 1 bis 5, in dem die Hydrierungsstufe (4) in Gegenwart mindestens eines Katalysators auf Nickelbasis, der auf einem Träger abgeschieden ist, durchgeführt wird.

7. Verfahren nach einem der Ansprüche 1 bis 6, in dem am Ende der Stufe (1) mindestens der Hauptteil (größere Teil) des Wassers, das durch die Dehydratation des Isopropylalkohols zu Propen in der Stufe (1) gebildet worden ist, eliminiert wird.

8. Verfahren nach einem der Ansprüche 1 bis 7, in dem jeder Katalysator in einem Fixbett angeordnet ist.

9. Verfahren zur Herstellung von reinem Phenol nach einem der Ansprüche 1 bis 8 ohne gleichzeitige Bildung von Aceton.

## Claims

1. A process for the production of phenol, characterised in that it comprises the following successive stages:

1) benzene is reacted with a charge containing propene and isopropyl alcohol in contact with at least one catalyst based on a dealuminated Y zeolite with a $SiO_2/Al_2O_3$ molar ratio of between 8 and 70, and the product obtained is then fractionated in such a way as to collect separately a first fraction containing non-converted benzene, a second fraction containing cumene and a third fraction containing at least one polyisopropylbenzene,

2) at least a part of said third fraction is reacted with benzene in contact with at least one catalyst based on a dealuminated Y zeolite with a $SiO_2/Al_2O_3$ molar ratio of between 8 and 70, and cumene is collected,

3) the cumene from stages 1) and 2) is transformed into a mixture of phenol and acetone, and then said mixture is fractionated in such a way as to collect phenol and acetone separately,

4) the acetone obtained at the end of stage 3) is at least partly hydrogenated into isopropyl alcohol which is then at least partly recycled to stage 1).

2. A process for the production of phenol, characterised in that it comprises the following successive stages:

1) benzene is reacted with a charge containing propene and isopropyl alcohol in contact with at least one catalyst based on a dealuminated Y zeolite with a $SiO_2/Al_2O_3$ molar ratio of between 8 and 70, and the product obtained is then fractionated in such a way as to collect separately a first fraction containing non-converted benzene, a second fraction containing cumene and a third fraction containing at least one polyisopropylbenzene,

2) at least a part of said third fraction is reacted with benzene in contact with at least one catalyst based on a dealuminated mordenite with a total Si/Al atomic ratio of between 20 and 60, and the cumene is collected,

3) the cumene from stages 1) and 2) is transformed into a mixture of phenol and acetone, and said mixture is then fractionated in such a way as to collect phenol and acetone separately,

4) the acetone obtained at the end of stage 3) is at least partly hydrogenated into isopropyl alcohol which is then recycled at least partly to stage 1).

3. A process according to one of Claims 1 and 2 wherein at least part of the benzene used in stage 2) is constituted of non-converted benzene coming from said first fraction obtained at the end of stage 1).

4. A process according to one of Claims 1 to 3 wherein the polyisopropylbenzenes not converted during stage 2) are at least partly recycled in said stage 2).

5. A process according to one of Claims 1 to 4, wherein the process whereby the cumene is transformed into a mixture of phenol and acetone during stage 3) comprises oxidation of the cumene and cumyl hydroperoxide, and then cleavage of said cumyl hydroperoxide into a mixture of phenol and acetone.

6. A process according to one of Claims 1 to 5, wherein stage 4) for hydrogenation takes place in the presence of at least one catalyst based on nickel deposited on a carrier.

7. A process according to one of Claims 1 to 6, wherein at the end of stage 1) at least the major part of the water formed by dehydration of the isopropyl alcohol into propene during stage 1) is removed.

8. A process according to one of Claims 1 to 7, wherein each catalyst is deposited in a fixed bed.

9. A process for the production of pure phenol according to one of Claims 1 to 8, without the co-production of acetone.

EP 0 434 485 B1

PL_unique